# EUROPÄISCHE PATENTSCHRIFT

(11) **EP 0 679 325 B1**
(45) Veröffentlichungstag und Bekanntmachung des Hinweises auf die Patenterteilung: **15.10.1997**
(21) Anmeldenummer: 94905056.1
(22) Anmeldetag: 13.01.1994
(51) Int. Cl.: H05H 3/04, G01N 15/10, G02B 21/32

(54) **VORRICHTUNG UND VERFAHREN ZUR HANDHABUNG, BEARBEITUNG UND BEOBACHTUNG KLEINER TEILCHEN, INSBESONDERE BIOLOGISCHER TEILCHEN**
DEVICE AND PROCESS FOR HANDLING, TREATING AND OBSERVING SMALL PARTICLES, ESPECIALLY BIOLOGICAL PARTICLES
DISPOSITIF ET PROCEDE PERMETTANT DE MANIPULER, DE TRAITER ET D'OBSERVER DE PETITES PARTICULES, NOTAMMENT DES PARTICULES BIOLOGIQUES

(30) Priorität: 13.01.1993 DE 4300698
(43) Veröffentlichungstag der Anmeldung: 02.11.1995
(73) Patentinhaber: SCHUTZE, Raimund, D-82515 Wolfratshausen (DE)
(72) Erfinder: SCHUTZE, Raimund, D-82515 Wolfratshausen (DE)
(74) Vertreter: Sajda, Wolf E., Dipl.-Phys.
(86) Internationale Anmeldenummer: EP9400090
(87) Internationale Veröffentlichungsnummer: WO9416543

(56) Entgegenhaltungen:
- EP-A- 0 517 454
- EP-A- 0 552 539
- PROCEEDINGS OF THE SPIE- LASER-TISSUE INTERACTION, Bd. 1202, 17. Januar 1990, Los Angeles, CA, US, Seiten 272-280; R.W. STEUBING et al.: 'Single Beam Optical Trapping and Micromanipulation of Mammalian Cells'
- NATURE, Bd. 330, Nr. 6150, 24. Dezember 1987, UK, Seiten 769-771; A. ASHKIN et al. 'Optical trapping and manipulation of single cells using infrared laser beams'
- BERICHTE BUNSENGESELLSCHAFT PHYSIKALISCHE CHEMIE, Bd. 93, 1989, Weinheim DE, Seiten 254-260; A. ASHKIN et al. 'Optical Trapping and Manipulation of Single Living Cells Using Infra-Red Laser Beams'
- PATENT ABSTRACTS OF JAPAN vol. 15, no. 413 (C-877) 22. Oktober 1991; & JP-A-03 172 167

## Beschreibung

Die Erfindung betrifft eine Vorrichtung zur Handhabung, Bearbeitung und Beobachtung kleiner Teilchen, insbesondere biologischer Teilchen, umfassend mindestens einen ersten Laser, der Lichtstrahlen in einem ersten Wellenlängenbereich erzeugt, die mit einer ersten optischen Einrichtung mit ausreichender Konvergenz fokussiert werden, um in einem vorgegebenen Bereich eine optische Falle zu bilden; einen Objektträger zur Aufnahme von Teilchen, insbesondere biologischen Teilchen; eine Lichtquelle für Beobachtungslicht; und Beobachtungs- und Aufzeichnungseinrichtungen, um Teilchen in dem Objektträger zu beobachten und ihr Verhalten aufzuzeichnen.

Die Erfindung betrifft weiterhin ein Verfahren zur Handhabung, Bearbeitung und Beobachtung kleiner Teilchen, insbesondere biologischer Teilchen, bei dem die Objekte in einem Objektträger mit mindestens einem ersten Laser, der Lichtstrahlen in einem ersten Wellenlängenbereich erzeugt, in einer optischen Falle fixiert werden und die Objekte mit Beobachtungs- und Aufzeichnungseinrichtungen beobachtet werden und/oder das Verhalten der Objekte aufgezeichnet wird.

Eine derartige Vorrichtung ist beispielsweise aus der US-PS 4 893 886 bekannt, wobei mit einer sogenannten optischen Falle gearbeitet wird, die einen stark fokussierten Laserstrahl mit einem Intensitätsprofil mit annähernd Gauss'scher Verteilung verwendet. In diesen optischen Fallen werden die Komponenten der Strahlungsdruck-Streukraft und der Gradientenkraft miteinander kombiniert, um einen Punkt eines stabilen Gleichgewichtes zu bilden, der sich dicht bei dem Fokus des Laserstrahles befindet. Die Streukraft ist dabei proportional zur optischen Intensität und wirkt in der Richtung des einfallenden Laserlichtes. Die Gradientenkraft ist proportional zur optischen Intensität und zeigt in Richtung des Intensitätsgradienten.

Nähere Einzelheiten von derartigen optischen Fallen und die dazugehörigen physikalischen Grundlagen sind beispielsweise in der Veröffentlichung "Optical Trapping and Manipulation of Single Living Cells Using Infra-Red Laser Beams", A. Ashkin et al. in BERICHTE DER BUNSEN-GESELLSCHAFT FÜR PHYSIKALISCHE CHEMIE, März 1989, Seiten 254 bis 260 beschrieben.

Mit derartigen Vorrichtungen können kleine Teilchen, insbesondere biologische Teilchen, die sich in einer Flüssigkeit im Objektträger sonst frei bewegen können, eingefangen, festgehalten und manipuliert werden. Dabei tritt die Schwierigkeit auf, daß die Handhabung der Teilchen einerseits und ihre Beobachtung andererseits zur gleichen Zeit erfolgen sollte, um eine exakte Bearbeitung zu ermöglichen. Weiter ist aus EP-A-0 517 454 eine Vorrichtung und ein Verfahren gemäß Oberbegriff von Anspruch 1 und 12 bekannt.

Der Erfindung liegt daher die Aufgabe zugrunde, eine Vorrichtung und ein Verfahren anzugeben, mit denen eine gezielte und exakte Handhabung, Bearbeitung und Beobachtung von kleinen Teilchen, insbesondere biologischen Teilchen möglich ist.

Die erfindungsgemäße Lösung besteht aus einer Vorrichtung gemäß Anspruch 1 und aus einem Verfahren gemäß Anspruch 12.

In Weiterbildung der erfindungsgemäßen Vorrichtung ist vorgesehen, daß der jeweilige erste Laser ein gegebenenfalls in seiner Wellenlänge einstellbarer Laser, insbesondere ein IR-Laser ist und daß der jeweilige zweite Laser ein gegebenenfalls in seiner Wellenlänge einstellbarer UV-Laser, insbesondere ein gepulster UV-Laser ist. Auf diese Weise ist eine für praktische Zwecke geeignete Fixierung der jeweiligen Teilchen möglich, während die eigentliche Behandlung mit dem UV-Laser erfolgt, ohne daß die Gefahr besteht, daß zu große Energiemengen zugeführt werden, die sonst eine unerwünschte Beschädigung von Teilchen hervorrufen könnten.

Gemäß einer speziellen Ausführungsform der erfindungsgemäßen Vorrichtung ist vorgesehen, daß der jeweilige erste Laser ein Nd-YAG-Laser, ein Nd-YLF-Laser oder ein Titan-Saphir-Laser und der jeweilige zweite Laser ein Stickstofflaser, ein frequenzvervielfachter IR-Laser oder ein gepumpter Farbstofflaser ist.

In Weiterbildung der erfindungsgemäßen Vorrichtung ist vorgesehen, daß der jeweilige erste Laser und der jeweilige zweite Laser in demselben Turm angeordnet, aber unabhängig voneinander positionierbar und justierbar sind. Auf diese Weise ist eine Grundeinstellung der Lichtquellen in raumsparender Weise möglich, insbesondere dann, wenn die jeweiligen Laser auf entsprechenden Montageplatten übereinander angebracht sind. Die Komponenten der Fokussierungs- und Umlenkoptik können ihrerseits für eine kompakte Bauform auf einer gemeinsamen Montageplatte montiert sein, die dem Turm zugeordnet ist.

Die Lichtquellen, die Lichtstrahlen in den jeweiligen Wellenlängenbereichen erzeugen, können als separate Laser vorgesehen sein. Bei einer bevorzugten Ausführungsform der erfindungsgemäßen Vorrichtung ist vorgesehen, daß der Lichtstrahl des ersten Lasers mit einem Strahlteiler geteilt wird, der zumindest erste und zweite Lichtstrahlen in dem ersten Wellenlängenbereich erzeugt, die zumindest teilweise separat geführt und dann auf das Objekt im Objektträger gerichtet werden. Bei Bedarf können auch weitere Lichtstrahlen mit einem derartigen Strahlteiler von dem Lichtstrahl des ersten Lasers abgezweigt, zumindest teilweise separat geführt und dann auf das Objekt im Objektträger gerichtet werden, wenn mehrere solche Lichtstrahlen als optische Falle verwendet werden sollen.

Bei einer speziellen Ausführungsform der erfindungsgemäßen Vorrichtung ist vorgesehen, daß der Strahlteiler ein polarisierender Strahlteiler ist, der einen ersten Lichtstrahl mit s-polarisiertem Licht und einen zweiten Lichtstrahl mit p-polarisiertem Licht erzeugt und die Phasenlage zwischen diesen beiden Lichtstrahlen einstellt, und daß das prozentuale Verhältnis zwischen den Intensitäten der jeweiligen Lichtstrahlen in dem ersten Wellenlängenbereich einstellbar ist.

Bei der erfindungsgemäßen Vorrichtung erweist es sich als zweckmäßig, wenn für jeden der Lichtstrahlen von dem ersten Laser und dem zweiten Laser eine eigene Aufweitoptik vorgesehen ist, die jeweils dreidimensional, insbesondere in drei orthogonalen Achsenrichtungen einstellbar sind.

Weiterhin ist es bei der erfindungsgemäßen Vorrichtung zweckmäßig, wenn die im Strahlengang der Lichtstrahlen von dem ersten Laser und dem zweiten Laser vorgesehenen Spiegel und Strahlteiler unabhängig von den Aufweitoptiken drehbar bzw. kippbar sind. Damit bietet sich der Vorteil einer weiteren Einstellmöglichkeit der jeweiligen Lichtstrahlen in der x-y-Ebene.

In Weiterbildung der erfindungsgemäßen Vorrichtung ist vorgesehen, daß der Lichtstrahl des Beobachtungslichtes durch Einstellung des Objektivs und/oder des Objektträgers längs der optischen Achse in z-Richtung auf das Objekt im Objektträger fokussierbar ist und daß der Beobachtungsort für den Lichtstrahl des Beobachtungslichtes in der Objektebene durch Verstellen des Objektträgers innerhalb der Objektebene in einer x-y-Ebene einstellbar ist.

Weiterhin ist es zweckmäßig, wenn im Strahlengang des jeweiligen ersten Lasers und zweiten Lasers ein Strahlabschwächer vorgesehen ist, mit dem die Lichtstrahlen in dem jeweiligen Wellenlängenbereich in vorgegebenen Abstufungen oder kontinuierlich abgeschwächt werden, bevor sie auf das Objekt im Objektträger gerichtet werden. Damit ist eine gezielte Einstellung der Intensität dieser Lichtstrahlen möglich, um unerwünschte Beschädigungen von Teilchen zu vermeiden.

Besonders vorteilhaft ist es, wenn bei der erfindungsgemäßen Vorrichtung die Lichtstrahlen in dem ersten Wellenlängenbereich und die Lichtstrahlen in dem zweiten Wellenlängenbereich über einen gemeinsamen Spiegel durch ein gemeinsames Objektiv auf das jeweilige Objekt im Objektträger gerichtet werden. Auf diese Weise ist eine besonders kompakte Bauform der Vorrichtung möglich. Zugleich wird der Aufbau des Strahlenganges vereinfacht und ein zuverlässiger Betrieb der Vorrichtung gewährleistet.

Bei einer speziellen Ausführungsform der erfindungsgemäßen Vorrichtung ist vorgesehen, daß die von den Strahlerzeugungseinrichtungen erzeugten, anschließend behandelten, umgelenkten und auf das jeweilige Objekt fokussierten Lichtstrahlen alle im wesentlichen in derselben ersten Ebene liegen, daß der Objektträger sich in einer zweiten Ebene, senkrecht zur ersten Ebene befindet, und daß die Spiegel bzw. Strahlteiler zur Umlenkung der einzelnen Lichtstrahlen ebenfalls in Ebenen senkrecht zur ersten Ebene angeordnet sind. Damit steht eine besonders kompakte und leicht zu handhabende Vorrichtung zur Verfügung, die eine zuverlässige Koordinierung der jeweiligen Lichtstrahlen gewährleistet.

Mit dem erfindungsgemäßen Verfahren wird in vorteilhafter Weise erreicht, daß der Benutzer einer entsprechenden Einrichtung eine stabile Ausgangsstellung verwendet und sich orientieren kann, in welcher Ebene Vorgänge stattfinden bzw. beeinflußt werden.

In Weiterbildung des erfindungsgemäßen Verfahrens ist vorgesehen, daß ein in der optischen Falle des ersten Lasers gefangenes Teilchen (a) durch Verstellen von mindestens einem Lichtstrahl in dem ersten Wellenlängenbereich in x-y-Richtung und/oder (b) durch Verstellen des Objektträgers in x-y-Richtung in der Objektebene verschiebbar ist, wobei im Falle (a) nur das gefangene Teilchen und im Falle (b) sämtliche Teilchen bewegt werden.

Auch ist in Weiterbildung des erfindungsgemäßen Verfahrens vorgesehen, daß ein in der optischen Falle des jeweiligen ersten Lasers gefangenes Teilchen
a) durch Verstellen von mindestens einem Lichtstrahl in dem ersten Wellenlängenbereich in z-Richtung und/oder
b) durch Verstellen des Objektivs und/oder des Objektträgers in z-Richtung verschiebbar ist,
wobei im Falle a) das gefangene Teilchen aus der gewählten Beobachtungsebene heraus bewegt wird und im Falle b) das gefangene Teilchen in der gewählten Beobachtungsebene bleibt.

Selbstverständlich sind nicht nur Verschiebungen von Teilchen möglich, sondern es können in Weiterbildung des erfindungsgemäßen Verfahrens, bei Verwendung von mindestens zwei getrennten Lichtstrahlen in dem ersten Wellenlängenbereich, Drehungen eines Teilchens in der optischen Falle vorgenommen werden, nämlich dadurch, daß (a) der eine Lichtstrahl in seiner Ausgangsstellung bleibt und der andere Lichtstrahl eine Bewegung in x-y-Richtung ausführt, oder (b) der eine Lichtstrahl in seiner Ausgangsstellung bleibt und der andere Lichtstrahl eine Bewegung in z-Richtung ausführt, oder (c) zumindest zwei Lichtstrahlen entgegengesetzte Bewegungen oder unterschiedlich weite Bewegungen in z-Richtung ausführen, oder (d) Kombinationen der Bewegungen gemäß (a), (b) und (c) ausgeführt werden.

In Weiterbildung des erfindungsgemäßen Verfahrens ist vorgesehen, daß die Behandlung der Teilchen mit den Lichtstrahlen in dem zweiten Wellenlängenbereich in einer beliebig wählbaren x-y-Ebene des Objektträgers durchgeführt wird, wobei die Beobachtungsebene in derselben Ebene oder einer anderen, parallel dazu liegenden Ebene vorgenommen werden kann. Eine derartige Änderung der Beobachtungsebene ist ohne weiteres möglich, nachdem die Ausgangsstellung zu Beginn des Verfahrens eingenommen worden ist.

Zweckmäßigerweise werden bei dem erfindungsgemäßen Verfahren für die Fixierung der Teilchen in der optischen Falle sichtbare oder IR-Laserstrahlen und für die Behandlung der Teilchen UV-Laserstrahlen, insbesondere gepulste UV-Laserstrahlen verwendet.

Wenn in Weiterbildung des erfindungsgemäßen Verfahrens sämtliche Lichtstrahlen gleichzeitig durch dasselbe Objektiv auf das entsprechende Objekt im Objektträger gerichtet werden, so ist eine besonders zuverlässige Einstellung und Handhabung möglich.

Schließlich sind beim erfindungsgemäßen Verfahren sämtliche Lichtstrahlen zur Steuerung der Behandlung und/oder Beobachtung unabhängig voneinander in ihrer Intensität einstellbar und/oder einschaltbar bzw. ausschaltbar. Damit wird in vorteilhafter Weise erreicht, daß eine Vielzahl von Möglichkeiten für die Handhabung, Bearbeitung und Beobachtung von Teilchen gegeben ist.

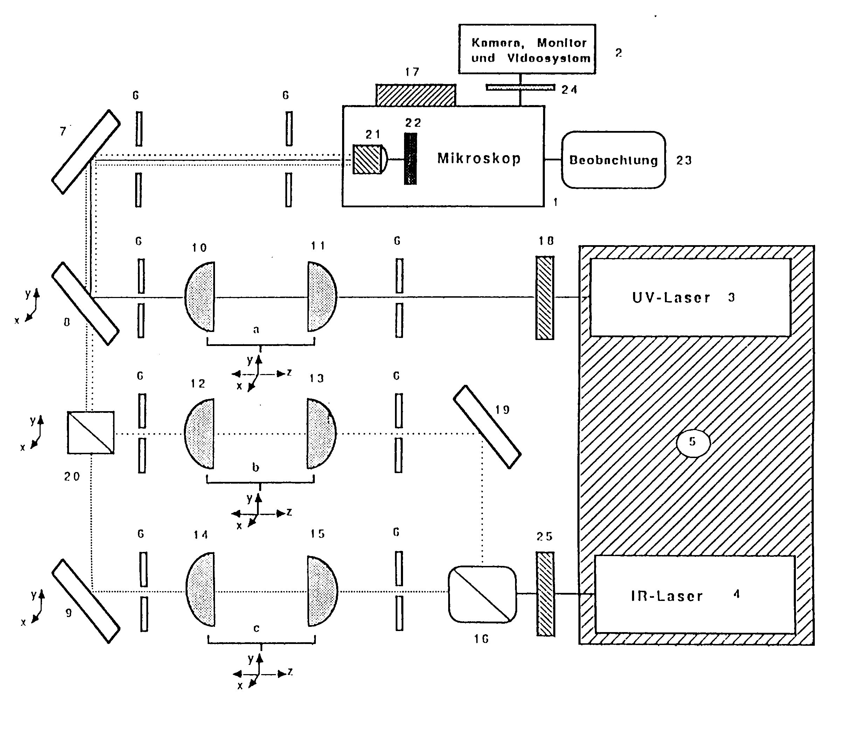

Die Erfindung wird nachstehend, auch hinsichtlich weiterer Merkmale und Vorteile, anhand der Beschreibung eines Ausführungsbeispiels und unter Bezugnahme auf die beiliegende Zeichnung näher erläutert.

Die einzige Figur der Zeichnung zeigt eine schematische Darstellung einer Ausführungsform der erfindungsgemäßen Vorrichtung mit den verwendeten Lichtquellen und Strahlengängen.

In der Zeichnung erkennt man einen gemeinsamen Laserturm 5, an dem ein IR-Laser 4 als erster Laser und ein UV-Laser 3 als zweiter Laser zweckmäßigerweise übereinander angebracht sind. Weiterhin können auf einer gemeinsamen Montageplatte mehrere Schienen mit zugeordneten Hubeinrichtungen für die entsprechenden optischen Komponenten angebracht sein, um diese relativ zu den beiden Lasern 3 und 4 zu justieren, derart, daß die von ihnen ausgehenden Lichtstrahlen in den jeweiligen Wellenlängenbereichen durch die nachgeschalteten optischen Einrichtungen zu dem Objektträger 22 gelangen. Zweckmäßigerweise werden dabei parallele Lichtstrahlen von den beiden Lasern 3 und 4 erzeugt. Die optischen Komponenten auf den jeweiligen Schienen können dabei zweckmäßigerweise in Modulbauweise vorgesehen sein.

Bei der dargestellten Ausführungsform erzeugt der IR-Laser 4 einen Lichtstrahl, der mit einem Strahlteiler 16 in einen ersten Lichtstrahl und einen zweiten Lichtstrahl geteilt wird. Der erste Lichtstrahl geht durch entsprechende Blenden 6 und eine Aufweitoptik 14, 15 hindurch und wird dann mit einem IR-reflektierenden Spiegel 9 umgelenkt und geht anschließend durch einen Strahlteiler, z.B. ein Prisma oder einen halbdurchlässigen Spiegel 20 sowie einen weiteren halbdurchlässigen Spiegel 8 oder ein entsprechendes Prisma hindurch. Er wird dann mit einem Umlenkspiegel 7 einem schematisch angedeuteten Mikroskop 1 zugeführt, das ein Objektiv 21, einen Objektträger 22 und eine Lichtquelle 17 für Beobachtungslicht aufweist.

Der abgezweigte Teil des Lichtstrahles vom IR-Laser 4 gelangt von dem Strahlteiler 16 zu einem Spiegel 19 und geht dann durch entsprechende Blenden und eine zweite Aufweitoptik 12, 13 hindurch. Anschließend wird dieser zweite Lichtstrahl mit dem Strahlteiler bzw. halbdurchlässigen Spiegel 20 umgelenkt und in gleicher Weise wie der erste Lichtstrahl von dem IR-Laser 4 zum Mikroskop 1 geführt.

Zur Erzeugung des zweiten Lichtstrahles in dem ersten Wellenlängenbereich kann selbstverständlich auch ein weiterer, nicht dargestellter IR-Laser verwendet werden. Dadurch bieten sich zwar zusätzliche Möglichkeiten für die Intensität, Polarisierung, Wellenlänge und Steuerbarkeit eines derartigen Laserstrahles, zugleich werden der Aufwand und die erforderlichen Kosten gesteigert, so daß eine problemorientierte Entscheidung in der Praxis zu treffen ist.

Bei der dargestellten Ausführungsform kann der Strahlteiler 16 ein einfacher Strahlteiler sein, so daß bei ausreichender Ausgangsleistung und Intensität des Lichtes von dem IR-Laser ein Teil der Intensität für den zweiten Lichtstrahl abgezweigt wird. Bei einer bevorzugten Ausführungsform wird jedoch als Strahlteiler 16 ein polarisierender Strahlteiler verwendet, der einen ersten Lichtstrahl mit s-polarisiertem Licht und einen zweiten Lichtstrahl mit p-polarisiertem Licht erzeugt und die Phasenlage zwischen diesen beiden Lichtstrahlen einstellt, wobei zugleich auch das prozentuale Verhältnis zwischen den Intensitäten der jeweiligen Lichtstrahlen in dem ersten Wellenlängenbereich des IR-Lasers 4 einstellbar ist.

Die in diesem Bereich verwendeten optischen Komponenten sind zweckmäßigerweise für Infrarot-Licht vergütet, um eine exakte Strahlführung ohne unerwünschte Verluste zu gewährleisten. Falls gewünscht, kann in dem Strahlengang des ersten und/oder des zweiten Lichtstrahles von dem IR-Laser 4 ein Strahlabschwächer 25 vorgesehen sein, um die Leistung der verwendeten Lichtstrahlen einzustellen. Alternativ oder zusätzlich ist es möglich, die Leistung des IR-Lasers 4 selbst in einem vorgegebenen Bereich einzustellen.

Unabhängig davon sind im Strahlengang des ersten und zweiten Lichtstrahles von dem IR-Laser 4 Mittel vorgesehen, mit denen der jeweilige Lichtstrahl unterbrochen werden kann. Dazu können entweder die Blenden 6 oder aber separate Schließeinrichtungen verwendet werden.
Als zweiter Laser ist ein UV-Laser 3 vorgesehen, dessen Lichtstrahl in einem zweiten Wellenlängenbereich durch einen Strahlabschwächer 18, Blenden 6 und eine eigene Aufweitoptik 10, 11 hindurchgeht und dann mit dem Spiegel 8 umgelenkt und über den weiteren Spiegel 7 zum Objektiv 21 und dem Objektträger 22 gelenkt wird. Der UV-Laser 3 ist zweckmäßigerweise ein gepulster UV-Laser, um die zugeführte Energie des UV-Lichtes präzise einstellen und steuern zu können, um Beschädigungen der zu behandelnden Objekte auszuschließen.

Zusätzlich ist der Strahlabschwächer 18 vorgesehen, mit dem die Lichtstrahlen in dem zweiten Wellenlängenbereich (UV-Licht) in vorgegebenen Abstufungen oder kontinuierlich abgeschwächt werden. Der Strahlabschwächer 18 kann somit ein einstellbares Filter oder ein Strahlteiler sein.

Die im Strahlengang des UV-Lasers 3 befindlichen Komponenten sind selbstverständlich für derartiges UV-Licht ausgelegt und geeignet, die Komponenten 7 und 8 sowie 21 sind sowohl für IR-Licht als auch für UV-Licht ausgelegt und entsprechend vergütet.

Dem Mikroskop 1 sind eine Lichtquelle 17 für Beobachtungslicht, also zweckmäßigerweise sichtbares Licht, sowie einerseits eine visuelle Beobachtungseinrichtung 23, zweckmäßigerweise mit einem entsprechenden Schutzfilter, und andererseits kombinierte Beobachtungs- und Aufzeichnungseinrichtungen 2 zugeordnet, die beispielsweise eine Kamera, einen Monitor sowie ein Videosystem umfassen. Dabei ist zweckmäßigerweise ein variables IR-Filter 24 dazwischengeschaltet, um eine entsprechende Schutzfunktion auszuüben.

Bei einer derartigen Vorrichtung können verschiedene Laser als Lichtquellen verwendet werden, die über eine kompakte Einkoppeloptik mit dem Mikroskop 1 verbunden sind. Sowohl der (gepulste) UV-Laser 3, der beispielsweise im nahen UV arbeitet, als auch der (kontinuierlich betriebene) IR-Laser 4, der beispielsweise im nahen IR arbeitet, müssen sehr gut (beugungsbegrenzt) fokussierbar sein, also eine minimale Strahldivergenz haben. Unter diesen Voraussetzungen können Laser unterschiedlichster Bauart verwendet werden, insbesondere sind kleine, kompakte Laser für Laboruntersuchungen oder dergleichen geeignet.

Beispielsweise kann als UV-Laser 3 ein Stickstofflaser oder ein frequenzvervielfachter IR-Laser verwendet werden, während als IR-Laser 4 ein diodengepumpter Nd-YAG-Laser oder Nd-YLF-Laser verwendet wird, deren Laserleistung entsprechend gewählt wird. Hierbei ist zu berücksichtigen, daß in Flüssigkeit bewegliche Teilchen ohne Eigenbewegung in dem Objektträger 22 bereits bei geringer Laserleistung eingefangen werden können, während Partikel mit Eigendynamik oder Partikel in hochviskosen Lösungen nur mit größeren Laserleistungen gefangen werden können. Wie bereits erwähnt, können für die Einkopplung von zwei unabhängig voneinander beweglichen IR-Laserstrahlen entweder zwei diodengepumpte Nd-YAG-Laser eingesetzt werden, oder aber ein blitzlampengepumpter Nd-YAG-Laser mit höherer Ausgangsleistung verwendet werden, wie es schematisch in der Zeichnung angedeutet ist.

Wichtig ist bei der erfindungsgemäßen Vorrichtung, daß für jeden Lichtstrahl von den jeweiligen Lasern 3 und 4 eine eigene Aufweitoptik 10, 11 bzw. 12, 13 bzw. 14, 15 vorgesehen ist, die jeweils dreidimensional, insbesondere in drei orthogonal zueinander stehenden Richtungen verstellbar sind, wie es schematisch in der Zeichnung angedeutet ist. Mit diesen Aufweitoptiken wird der jeweilige Lichtstrahl so aufgeweitet, daß der Strahldurchmesser die rückwärtige Apertur des Objektives 21 des Mikroskops 1 gerade ausfüllt bzw. leicht überfüllt. Beispielsweise können für diese Aufweitoptiken jeweils zwei plankonvexe Linsen oder aber eine plankonvexe und eine plankonkave Linse geeigneter Brennweite verwendet werden.

Soweit in der Zeichnung im Zusammenhang mit den Aufweitoptiken 10, 11 bzw. 12, 13 bzw. 14, 15 Koordinatenrichtungen angegeben sind, beziehen sich diese auf die Situation im Objektträger 22, wobei die x-y-Ebene senkrecht zur Zeichnungsebene verläuft und die z-Richtung orthogonal dazu in der Zeichnungsebene angesetzt ist.

Mit einer Verstellung von einer der Aufweitoptiken 12, 13 bzw. 14, 15 in der x-y-Ebene kann somit der Ort der Fixierung der optischen Falle in der x-y-Ebene verändert werden, während eine Bewegung in der z-Richtung eine Änderung der Fokussierung senkrecht zu der x-y-Ebene bedeutet, so daß das jeweilige in der optischen Falle gefangene Teilchen aus der (ursprünglichen) Beobachtungsebene herausbewegt wird.

Entsprechendes gilt für die Wirkungsweise der Aufweitoptik 10, 11 für den UV-Laser 3. Durch Veränderung dieser Aufweitoptik 10, 11 in x-y-Richtung ändert sich der Ort, an dem eine Beeinflussung von Teilchen in der Objektebene erfolgt. Eine Bewegung in z-Richtung bewirkt eine Defokussierung gegenüber der Beobachtungsebene in dieser Richtung. Somit kann die Bearbeitung wahlweise in der Fokus-Beobachtungsebene oder außerhalb dieser Fokus-Beobachtungsebene durchgeführt werden.

Zusätzlich oder alternativ ist es möglich, die Komponenten 8, 9 und 20 in Ebenen quer zur Zeichnungsebene zu drehen bzw. zu kippen, um die jeweiligen Lichtstrahlen in der x-y-Ebene zu bewegen.

Der Objektträger 22 ist in an sich bekannter Weise in den Richtungen der x-Achse, der y-Achse und der z-Achse bewegbar, um eine geeignete Einstellung vorzunehmen. Das Objektiv 21 ist seinerseits zumindest in z-Richtung verstellbar.

Bei der dargestellten Ausführungsform sind die Beobachtungs-und Aufzeichnungseinrichtungen so angeordnet, daß mit Durchlicht gearbeitet wird. Die Anordnung kann selbstverständlich auch so angeordnet sein, daß diese Beobachtungs- und Aufzeichnungseinrichtungen 2 bzw. 23 auf der einen Seite des Mikroskops 1 angeordnet sind und die Lichtquelle 17 für Beobachtungslicht in Strahlrichtung hinter dem Mikroskop 1 liegt, so daß dann mit Auflicht gearbeitet werden kann.

Mit der vorstehend beschriebenen Vorrichtung kann eine Vielzahl von Bearbeitungen und Handhabungen von kleinen Teilchen durchgeführt werden, die auch gleichzeitig zu beobachten sind. Das Festhalten von Teilchen an einem oder mehreren Orten erfolgt durch Einschalten bzw. Ausschalten der beiden IR-Laserstrahlen von dem IR-Laser 4, wobei die Anzahl dieser Lichtstrahlen gegebenenfalls auch vergrößert werden kann, indem die Vorrichtung in analoger Weise weitergebildet wird und beispielsweise von dem Strahlteiler 16 oder dem Strahlteiler 19 weitere Teilstrahlen abgezweigt und jeweils über eine eigene Aufweitoptik in das System eingekoppelt werden. Nachdem die Lichtstrahlen in dem ersten Wellenlängenbereich, also vom IR-Laser 4, die Lichtstrahlen in dem zweiten Wellenlängenbereich, also von dem UV-Laser 3, und die Lichtstrahlen des Beobachtungslichtes zu Beginn der Handhabung und Beobachtung unabhängig von ihren Wellenlängen in derselben Objektebene, also einer bestimmten x-y-Ebene des Objektträgers fokussiert worden sind, können die einzelnen Strahlen unabhängig voneinander beeinflußt werden, um Bewegungen der Teilchen in dem Objektträger 22 zu erreichen und diese ganz gezielt an einer gewünschten Stelle ihrer dreidimensionalen Ausdehnung zu bearbeiten, beispielsweise unter Verwendung des oben beschriebenen UV-Lasers 3.

Dabei können Bewegungen in der x-y-Ebene realisiert werden entweder durch Bewegung des Objektträgers 22 in x-y-Richtung, wobei dann alle darin vorhandenen Teilchen gleichzeitig in dieser Ebene bewegt werden. Andererseits kann eine Bewegung in dieser x-y-Richtung erfolgen durch Betätigen von mindestens einer der Aufweitoptiken 12, 13 bzw. 14, 15 oder durch Kippen von mindestens einer Spiegelfläche der Komponenten 8, 9, 20, so daß einzelne Teilchen unabhängig voneinander bewegt werden können.

Bewegungen in der z-Richtung können in unterschiedlicher Weise realisiert werden, einmal durch Bewegen des Objektträgers 22 in z-Richtung relativ zum Objektiv 21, zum anderen durch Bewegen des Objektives 21 in z-Richtung relativ zum Objektträger. In beiden Fällen bleibt die Fokussierung für das sichtbare Beobachtungslicht erhalten.

Unabhängig davon kann zumindest eine der Aufweitoptiken 12, 13 und 14, 15 in z-Richtung betätigt werden, so daß ein Kippen bzw. Drehen eines Objektes in dem Objektträger 22 möglich ist. Auf diese Weise kann ein dreidimensionales Objekt in verschiedenen gedachten Schnittebenen des Objektes beobachtet und bearbeitet werden.
Da die Aufweitoptik 10, 11 für den UV-Laser 3 unabhängig davon bewegbar ist, kann die Bearbeitung wahlweise in der Fokus-Beobachtungsebene oder außerhalb der Fokus-Beobachtungsebene durchgeführt werden.

Mit der vorstehend beschriebenen Vorrichtung ist ein Positionieren eines Objektes unter Verwendung der IR-Laserstrahlen in einer anderen Ebene als der Objektträgerebene möglich, ohne daß das Objekt an dem Objektträger "festgeklemmt" zu werden braucht, denn die Fixierung erfolgt lediglich mit den jeweiligen IR-Laserstrahlen, die von dem IR-Laser 4 geliefert werden. Damit ist die (gleichzeitige) Beobachtung und Bearbeitung (auch von mehreren) frei beweglichen Objekten in einfacher und zuverlässiger Weise möglich.

Bei der vorstehend beschriebenen Ausführungsform wird eine Anordnung verwendet, bei der sämtliche Lichtstrahlen, die von den jeweiligen Lichtquellen erzeugt werden, von den Lichterzeugungseinrichtungen aus praktisch in derselben Ebene geführt, umgelenkt und fokussiert werden. Die Zuführung der verschiedenen Lichtstrahlen zum Objektträger 22 erfolgt dabei über einen gemeinsamen Umlenkspiegel 7 sowie das Objektiv 21. Auf diese Weise ist eine besonders kompakte Anordnung möglich, die zugleich eine zuverlässige Funktion der Vorrichtung gewährleistet.

Selbstverständlich ist auch eine dreidimensionale Verteilung der verschiedenen Komponenten der optischen Einrichtungen möglich, dann wird es allerdings in den meisten Fällen erforderlich, sphärische Spiegel oder Parabolspiegel zu verwenden, um die jeweiligen Strahlen in der gewünschten Weise auf den Objektträger 22 zu richten. Dies kann für bestimmte Anwendungen zweckmäßig oder wünschenswert sein, wenn die kompakte Bauform nicht im Vordergrund steht.

## Patentansprüche

1. Vorrichtung zur Handhabung, Bearbeitung und Beobachtung kleiner Teilchen, insbesondere biologischer Teilchen, umfassend
- mindestens einen ersten Laser (4), der Lichtstrahlen in einem ersten Wellenlängenbereich erzeugt, die mit einer ersten optischen Einrichtung (12, 13; 14, 15; 21) mit ausreichender Konvergenz fokussiert werden, um in einem vorgegebenen Bereich eine optische Falle zu bilden,
- einen Objektträger (22) zur Aufnahme von Teilchen, insbesondere biologischen Teilchen,
- eine Lichtquelle (17) für Beobachtungslicht und
- Beobachtungs- und Aufzeichnungseinrichtungen (1, 2, 23), um Teilchen in dem Objektträger (22) zu beobachten und ihr Verhalten aufzuzeichnen, und
- mindestens einen zweiten Laser (3), der Lichtstrahlen in einem zweiten Wellenlängenbereich erzeugt, die mit einer zweiten optischen Einrichtung (10, 11; 21) mit ausreichender Konvergenz fokussiert werden, um im Bereich des Objektträgers (22) vorhandene Teilchen zu behandeln,
dadurch gekennzeichnet,
daß für jeden der Lichtstrahlen von dem ersten Laser (4) und dem zweiten Laser (3) eine eigene Aufweitoptik (12, 13; 14, 15; 10, 11) vorgesehen ist, die jeweils dreidimensional, insbesondere in drei orthogonalen Achsenrichtungen (x, y, z) einstellbar sind, derart,
daß die optischen Einrichtungen (10, 11; 12 bis 15; 21) für die Lichtstrahlen in dem ersten Wellenlängenbereich, für die Lichtstrahlen in dem zweiten Wellenlängenbereich und für die Lichtstrahlen des Beobachtungslichtes jeweils separat und unabhängig voneinander positionierbar und fokussierbar sind
und daß die Lichtstrahlen in dem ersten Wellenlängenbereich, die Lichtstrahlen in dem zweiten Wellenlängenbereich und die Lichtstrahlen des Beobachtungslichtes zu Beginn der Handhabung und Beobachtung unabhängig von ihren Wellenlängen in derselben Objektebene (x-y-Ebene) des Objektträgers (22) fokussiert sind, wobei die einzelnen Strahlen unabhängig voneinander beeinflußt werden können, um Bewegungen der Teilchen in dem Objektträger (22) zu erreichen und diese ganz gezielt an einer gewünschten Stelle ihrer dreidimensionalen Ausdehnung bearbeiten zu können, und wobei die Fokussierung für das sichtbare Beobachtungslicht erhalten bleiben kann.

2. Vorrichtung nach Anspruch 1,
dadurch gekennzeichnet,
daß der jeweilige erste Laser (4) ein gegebenenfalls in seiner Wellenlänge einstellbarer Laser, insbesondere ein IR-Laser ist
und daß der jeweilige zweite Laser (3) ein gegebenenfalls in seiner Wellenlänge einstellbarer UV-Laser, insbesondere ein gepulster UV-Laser ist.

3. Vorrichtung nach Anspruch 1 oder 2,
dadurch gekennzeichnet,
daß der jeweilige erste Laser (4) ein Nd-YAG-Laser, ein Nd-YLF-Laser oder ein Titan-Saphir-Laser und der jeweilige zweite Laser (3) ein Stickstofflaser, ein frequenzvervielfachter IR-Laser oder ein gepumpter Farbstofflaser ist.

4. Vorrichtung nach einem der Ansprüche 1 bis 3,
dadurch gekennzeichnet,
daß der jeweilige erste Laser (4) und der jeweilige zweite Laser (3) in demselben Turm (5) angeordnet, aber unabhängig voneinander positionierbar und justierbar sind.

5. Vorrichtung nach einem der Ansprüche 1 bis 4,
dadurch gekennzeichnet,
daß der Lichtstrahl des ersten Lasers (4) mit einem Strahlteiler (16) geteilt wird, der zumindest erste und zweite Lichtstrahlen in dem ersten Wellenlängenbereich erzeugt, die zumindest teilweise separat geführt und dann auf das Objekt im Objektträger (22) gerichtet werden.

6. Vorrichtung nach Anspruch 5,
dadurch gekennzeichnet,
daß der Strahlteiler (16) ein polarisierender Strahlteiler ist, der einen ersten Lichtstrahl mit s-polarisiertem Licht und einen zweiten Lichtstrahl mit p-polarisiertem Licht erzeugt und die Phasenlage zwischen diesen beiden Lichtstrahlen einstellt,
und daß das prozentuale Verhältnis zwischen den Intensitäten der jeweiligen Lichtstrahlen in dem ersten Wellenlängenbereich einstellbar ist.

7. Vorrichtung nach einem der Ansprüche 1 bis 6,
dadurch gekennzeichnet,
daß die im Strahlengang der Lichtstrahlen von dem ersten Laser (4) und dem zweiten Laser (3) vorgesehenen Spiegel und Strahlteiler (8, 9, 20) unabhängig von den Aufweitoptiken drehbar bzw. kippbar sind.

8. Vorrichtung nach einem der Ansprüche 1 bis 7,
dadurch gekennzeichnet,
daß der Lichtstrahl des Beobachtungslichtes durch Einstellung des Objektivs (21) und/oder des Objektträgers (22) längs der optischen Achse (z-Richtung) auf das Objekt im Objektträger (22) fokussierbar ist
und daß der Beobachtungsort für den Lichtstrahl des Beobachtungslichtes in der Objektebene (x-y-Ebene) durch Verstellen des Objektträgers (22) innerhalb der Objektebene in einer x-y-Ebene einstellbar ist.

9. Vorrichtung nach einem der Ansprüche 1 bis 8,
dadurch gekennzeichnet,
daß im Strahlengang des jeweiligen ersten Lasers (4) und zweiten Lasers (3) ein Strahlabschwächer (18, 25) vorgesehen ist, mit dem die Lichtstrahlen in dem jeweiligen Wellenlängenbereich in vorgegebenen Abstufungen oder kontinuierlich abgeschwächt werden, bevor sie auf das Objekt im Objektträger (22) gerichtet werden.

10. Vorrichtung nach einem der Ansprüche 1 bis 9,
dadurch gekennzeichnet,
daß die Lichtstrahlen in dem ersten Wellenlängenbereich und die Lichtstrahlen in dem zweiten Wellenlängenbereich über einen gemeinsamen Spiegel (7) durch ein gemeinsames Objektiv (21) auf das jeweilige Objekt im Objektträger (22) gerichtet werden.

11. Vorrichtung nach einem der Ansprüche 1 bis 10,
dadurch gekennzeichnet,
daß die von den Strahlerzeugungseinrichtungen erzeugten, anschließend behandelten, umgelenkten und auf das jeweilige Objekt fokussierten Lichtstrahlen alle im wesentlichen in derselben ersten Ebene liegen,
daß der Objektträger (22) sich in einer zweiten Ebene (x-y-Ebene) senkrecht zur ersten Ebene befindet
und daß die Spiegel bzw. Strahlteiler (7, 8, 9, 19, 20) zur Umlenkung der einzelnen Lichtstrahlen ebenfalls in Ebenen im wesentlichen senkrecht zur ersten Ebene angeordnet sind.

12. Verfahren zur Handhabung, Bearbeitung und Beobachtung kleiner Teilchen, insbesonder biologischer Teilchen, bei dem die Objekte in einem Objektträger (22) mit mindestens einem ersten Laser (4), der Lichtstrahlen in einem ersten Wellenlängenbereich erzeugt, in einer optischen Falle fixiert werden und die Objekte mit Beobachtungs- und Aufzeichnungseinrichtungen (1, 2, 23) beobachtet werden und/oder das Verhalten der Objekte aufgezeichnet wird, und bei dem mindestens ein zweiter Laser (3) verwendet wird, der Lichtstrahlen in einem zweiten Wellenlängenbereich erzeugt, die mit ausreichender Konvergenz fokussiert werden, um im Bereich des Objektträgers (22) vorhandene Teilchen zu behandeln,
dadurch gekennzeichnet,
daß für jeden der Lichtstrahlen von dem ersten Laser (4) und dem zweiten Laser (3) eine eigene Aufweitoptik (12, 13; 14, 15; 10, 11) verwendet wird, die jeweils dreidimensional, insbesondere in drei orthogonalen Achsenrichtungen (x, y, z) einstellbar sind, derart,
daß die Lichtstrahlen in dem ersten Wellenlängenbereich, die Lichtstrahlen in dem zweiten Wellenlängenbereich und die Lichtstrahlen eines Beobachtungslichtes jeweils unabhängig voneinander mit separaten optischen Einrichtungen in der Objektebene (x-y-Ebene) einstellbar und in Achsenrichtung (z-Richtung) fokussierbar sind
und daß die Lichtstrahlen in dem ersten Wellenlängenbereich, die Lichtstrahlen in dem zweiten Wellenlängenbereich und die Lichtstrahlen des Beobachtungslichtes zu Beginn der Handhabung und Beobachtung unabhängig von ihren Wellenlängen in derselben Objektebene (x-y-Ebene) des Objektträgers (22) fokussiert werden, wobei die einzelnen Strahlen unabhängig voneinander beeinflußt werden können, um Bewegungen der Teilchen in dem Objektträger (22) zu erreichen und diese ganz gezielt an einer gewünschten Stelle ihrer dreidimensionalen Ausdehnung bearbeiten zu können, und wobei die Fokussierung für das sichtbare Beobachtungslicht erhalten bleiben kann.

13. Verfahren nach Anspruch 12,
dadurch gekennzeichnet,
daß ein in der optischen Falle des jeweiligen ersten Lasers (4) gefangenes Teilchen
a) durch Verstellen von mindestens einem Lichtstrahl in dem ersten Wellenlängenbereich in x-y-Richtung und/oder
b) durch Verstellen des Objektträgers (22) in x-y-Richtung in der Objektebene verschiebbar ist,
wobei im Falle a) nur das gefangene Teilchen und im Falle b) sämtliche Teilchen, ausgenommen das gefangene Teilchen, bewegt werden.

14. Verfahren nach Anspruch 12 oder 13,
dadurch gekennzeichnet,
daß ein in der optischen Falle des jeweiligen ersten Lasers (4) gefangenes Teilchen
a) durch Verstellen der Fokuslage von mindestens einem Lichtstrahl in dem ersten Wellenlängenbereich in z-Richtung und/oder
b) durch Verstellen des Objektivs (21) und/oder des Objektträgers (22) in z-Richtung verschiebbar ist,
wobei im Falle a) das gefangene Teilchen aus der gewählten Beobachtungsebene heraus bewegt wird und im Falle b) das gefangene Teilchen in der gewählten Beobachtungsebene bleibt.

15. Verfahren nach einem der Ansprüche 12 bis 14,
dadurch gekennzeichnet,
daß bei Verwendung von mindestens zwei getrennten Lichtstrahlen in dem ersten Wellenlängenbereich eine Drehung eines Teilchens in der optischen Falle dadurch erfolgt, daß
a) der eine Lichtstrahl in seiner Ausgangsstellung bleibt und der andere Lichtstrahl eine Bewegung in x-y-Richtung ausführt, oder
b) der eine Lichtstrahl in seiner Ausgangsstellung bleibt und der andere Lichtstrahl eine Bewegung in z-Richtung ausführt, oder
c) zumindest zwei Lichtstrahlen entgegengesetzte Bewegungen oder unterschiedlich weite Bewegungen in z-Richtung ausführen, oder
d) Kombinationen der Bewegungen gemäß a), b) und c) ausgeführt werden.

16. Verfahren nach einem der Ansprüche 12 bis 15,
dadurch gekennzeichnet,
daß die Behandlung der Teilchen mit den Lichtstrahlen in dem zweiten Wellenlängenbereich in einer beliebig wählbaren x-y-Ebene des Objektträgers (22) durchgeführt wird,
wobei die Beobachtungsebene in derselben Ebene oder einer anderen, parallel dazu liegenden Ebene vorgenommen werden kann.

17. Verfahren nach einem der Ansprüche 12 bis 16,
dadurch gekennzeichnet,
daß für die Fixierung der Teilchen in der optischen Falle sichtbare oder IR-Laserstrahlen und für die Behandlung der Teilchen UV-Laserstrahlen, insbesondere gepulste UV-Laserstrahlen verwendet werden.

18. Verfahren nach einem der Ansprüche 12 bis 17,
dadurch gekennzeichnet,
daß sämtliche Lichtstrahlen gleichzeitig durch dasselbe Objektiv (21) auf das entsprechende Objekt im Objektträger (22) gerichtet werden können.

19. Verfahren nach einem der Ansprüche 12 bis 18,
dadurch gekennzeichnet,
daß sämtliche Lichtstrahlen zur Steuerung der Behandlung und/oder Beobachtung unabhängig voneinander in ihrer Intensität einstellbar und/oder einschaltbar bzw. ausschaltbar sind.

## Claims

1. Apparatus for the manipulation, processing and observation of small particles, in particular biological particles, comprising
- at least one first laser (4) that generates light beams in a first wavelength range, which are focused with sufficient convergence by means of a first optical device (12, 13; 14, 15; 21) to form an optical trap in a predetermined region,
- an object holder (22) to contain particles, in particular biological particles,
- a light source (17) for observation light, and
- observation and recording devices (1, 2, 23) with which to observe particles in the object holder (22) and to record their behavior, and
- at least one second laser (3) that generates light beams in a second wavelength range, which are focused with sufficient convergence by means of a second optical device (10, 11; 21) to manipulate particles present in the region of the object holder (22),
characterized in that
for each of the beams from the first laser (4) and the second laser (3) its own diverging optic (12, 13; 14, 15; 10, 11) is provided, which in each case is adjustable in three dimensions, in particular along three orthogonal coordinates (x, y, z) in such a manner
that the optical devices (10, 11; 12 to 15; 21) for the beams in the first wavelength range, for the beams in the second wavelength range, and for the beams of the observation light can each be positioned and focused separately and independently of one another;
and in that the beams in the first wavelength range, the beams in the second wavelength range and the beams of the observation light are focused at the beginning of manipulation and observation in the same object plane (x-y plane) of the object holder (22), regardless of their wavelengths,
wherein the respective beams can be influenced independently of one another in order to achieve movements of the particles within the object holder (22) and to process them at a specifically targeted position within their three-dimensional extent, and wherein the focusing can be preserved for the visible observation light.

2. Apparatus as claimed in Claim 1,
characterized in that each of the first lasers (4) is a laser with wavelength that can be adjusted where appropriate, in particular an IR laser,
and in that each of the second lasers (3) is a UV laser with wavelength that can be adjusted where appropriate, in particular a pulsed UV laser.

3. Apparatus as claimed in Claim 1 or 2,
characterized in that each of the first lasers (4) is an Nd:YAG laser, an Nd:YLF laser or a titanium-sapphire laser, and in that each of the second lasers (3) is a nitrogen laser, a frequency-multiplied IR laser or a pumped dye laser.

4. Apparatus as claimed in any one of Claims 1 to 3,
characterized in that all of the first lasers (4) and the second lasers (3) are arranged in the same tower (5) but can be positioned and adjusted independently of one another.

5. Apparatus as claimed in any one of Claims 1 to 4,
characterized in that the light beam of the first laser (4) is divided by a beam splitter (16) that produces at least first and second beams in the first wavelength range, which pass separately over at least part of their paths and are then directed onto the object in the object holder (22).

6. Apparatus as claimed in Claim 5,
characterized in that the beam splitter (16) is a polarizing beam splitter that generates a first beam of s-polarized light and a second beam of p-polarized light and adjusts the phase shift between these two beams,
and in that the percentage relationship between the intensities of the individual beams in the first wavelength range is adjustable.

7. Apparatus as claimed in any one of Claims 1 to 6,
characterized in that the mirrors and beam splitters (8, 9, 20) provided in the path of the beams from the first laser (4) and the second laser (3) can be rotated or tilted independently of the diverging optics.

8. Apparatus as claimed in any one of Claims 1 to 7,
characterized in that the beam of the observation light can be focused on the object in the object holder (22) by adjusting the objective (21) and/or the object holder (22) along the optical axis (z direction);
and in that the site of observation for the beam of the observation light in the object plane (x-y plane) can be adjusted by adjusting the object holder (22) in an x-y plane within the object plane.

9. Apparatus as claimed in any one of Claims 1 to 8,
characterized in that in the path of the beam from each of the first lasers (4) and second lasers (3) a beam attenuator (18, 25) is provided, with which the beams in the associated wavelength range can be attenuated, in preset steps or continuously, before they are directed onto the object in the object holder (22).

10. Apparatus as claimed in any one of Claims 1 to 9,
characterized in that the light beams in the first wavelength range and the light beams in the second wavelength range are directed onto the relevant object in the object holder (22) through a common objective (21) by way of a common mirror (7).

11. Apparatus as claimed in any one of Claims 1 to 10,
characterized in that the light beams generated by the beam-generating devices and subsequently treated, deflected and focused on the relevant object all lie in substantially the same first plane;
in that the object holder (22) is positioned in a second plane (x-y plane) perpendicular to the first plane;
and in that the mirrors or beam splitters (7, 8, 9, 19, 20) to deflect the individual light beams are likewise arranged in planes substantially perpendicular to the first plane.

12. A method for the manipulation, processing and observation of small particles, in particular biological particles, in which the objects in an object holder (22) are fixed in an optical trap by means of at least one first laser (4) that generates light beams in a first wavelength range and the objects are observed and/or their behavior is recorded with observation and recording devices (1, 2, 23), and in which at least one second laser (3) is used, which generates light beams in a second wavelength range that are focused with sufficient convergence to manipulate particles present in the region of the object holder (22),
characterized in that for each of the beams from the first laser (4) and the second laser (3) its own diverging optic (12, 13; 14, 15; 10, 11) is used, which in each case is adjustable in three dimensions, in particular along three orthogonal coordinates (x, y, z) in such a manner that the light beams in the first wavelength range, the light beams in the second wavelength range and the beams of the observation light can each be adjusted in the object plane (x-y plane) and focused in the axial direction (z direction) with separate optical devices, independently of one another;
and in that the beams in the first wavelength range, the beams in the second wavelength range and the beams of the observation light are focused at the beginning of manipulation and observation in the same object plane (x-y plane) of the object holder (22), regardless of their wavelengths,
wherein the respective beams can be influenced independently of one another in order to achieve movements of the particles within the object holder (22) and to process them at a specifically targeted position within their three-dimensioned extent, wherein the focusing can be preserved for the visible observation light.

13. Method as claimed in Claim 12,
characterized in that a particle captured in the optical trap of each of the first lasers (4) can be shifted within the object plane
(a) by displacing at least one light beam in the first wavelength range in the x-y direction and/or
(b) by displacing the object holder (22) in the x-y direction in the object plane,
whereby in case (a) only the captured particle is moved and in case (b) all particles except for the captured particle are moved.

14. Method as claimed in Claim 12 or Claim 13,
characterized in that a particle captured in the optical trap of each of the first lasers (4) can be shifted (a) by displacing the focus position of at least one light beam in the first wavelength range in the z direction and/or (b) by displacing the objective (21) and/or the object holder (22) in the z direction,
whereby in case (a) the captured particle is moved out of the chosen observation plane and in case (b) the captured particle remains in the chosen observation plane.

15. Method as claimed in any one of Claims 12 to 14,
characterized in that when at least two separate light beams in the first wavelength range are used, rotation of a particle in the optical trap is brought about
(a) by leaving one beam in its starting position and moving the other beam in the x-y direction, or
(b) by leaving one beam in its starting position and moving the other beam in the z direction, or
(c) by moving at least two light beams in opposite directions or over different distances along the z axis, or
(d) by combinations of the movements according to (a), (b) and (c).

16. Method as claimed in any one of Claims 12 to 15,
characterized in that the manipulation of the particles with the light beams in the second wavelength range is carried out in an arbitrarily selectable x-y plane of the object holder (22), and the observation plane can be situated in the same plane or another plane parallel to it.

17. Method as claimed in any one of Claims 12 to 16,
characterized in that to fix the particles in the optical trap visible or IR laser beams are used and for manipulation of the particles UV laser beams, in particular pulsed UV laser beams, are used.

18. Method as claimed in any one of Claims 12 to 17,
characterized in that all the light beams can be directed simultaneously onto the relevant object in the object holder (22) through the same objective (21).

19. Method as claimed in any one of Claims 12 to 18,
characterized in that all the light beams for controlling the manipulation and/or observation can be adjusted in their intensity and/or be turned on and off independently of one another.

## Revendications

1. Dispositif permettant de manipuler, de traiter et d'observer de petites particules, notamment des particules biologiques, comprenant
- au moins un premier laser (4) produisant des rayons lumineux dans une première plage de longueurs d'ondes pouvant être focalisés avec suffisamment de convergences avec un premier dispositif optique (12,13 ; 14,15 ; 21) pour former un piège optique dans une zone prédéterminée,
- un support (22) d'objets pour recevoir les particules, notamment les particules biologiques,
- une source lumineuse (17) pour une lumière d'observation et
- des dispositifs d'observation et d'enregistrement (1,2,23) pour observer les petites particules sur le support (22) d'objets et en enregistrer leur comportement, et
- au moins un deuxième laser (3) générant des rayons lumineux dans une seconde plage de longueurs d'ondes pouvant être focalisés avec suffisamment de convergences par un deuxième dispositif optique (10,11 ; 21) afin de pouvoir traiter les petites particules situées dans la zone du support (22) d'objets
caractérisé en ce qu'il est prévu pour chaque rayon lumineux du premier (4) et second (3) laser sa propre optique de grossissement (12,13 ; 14,15 ; 10,11) qui chacune peut être réglable en trois dimensions, notamment dans les trois axes orthogonaux (x, y, z), de telle sorte que les dispositifs optiques (10,11; 12 à 15 ; 21) pour les rayons lumineux de la première plage de longueurs d'ondes, pour les rayons lumineux de la seconde plage de longueurs d'ondes et pour les rayons lumineux de la lumière d'observation sont chacun réglable en position et en focalisation de manière séparée et indépendamment des uns des autres et en ce que les rayons lumineux de la première plage de longueurs d'ondes, les rayons lumineux de la seconde plage de longueurs d'ondes, et les rayons lumineux de la lumière d'observation sont focalisés au début de la manipulation et de l'observation dans le même plan objet (plan x-y) du support (22) d'objets indépendamment de leur longueurs d'ondes, focalisation dans laquelle chaque rayonnement peut être influencé indépendamment des autres afin d'obtenir le mouvement des petites particules dans le support (22) d'objets et afin de pouvoir travailler celle-ci de manière très ciblée en une position souhaitée de leur enveloppe tridimensionnelle, et dans laquelle la focalisation de la lumière visible d'observation peut rester maintenue.

2. Dispositif selon la revendication 1, caractérisé en ce que le premier laser correspondant (4) est un laser le cas échéant réglable en sa longueur d'ondes, notamment un laser IR et en ce que le second laser correspondant (3) est un laser UV le cas échéant réglable en sa longueur d'ondes, notamment un laser UV pulsé.

3. Dispositif selon l'une des revendications 1 ou 2, caractérisé en ce que le premier laser correspondant (4) est un laser Nd-YAG, un laser Nd-YLF ou un laser-saphir-titan et que le second laser correspondant (3) est un laser à azote, un laser IR pluri-fréquentiel ou un laser de couleur pulsée.

4. Dispositif selon l'une des revendications 1 à 3, caractérisé en ce que le premier laser correspondant (4) et le second laser correspondant (3) sont agencés dans la même tour (5) mais sont réglables en position et ajustables indépendamment l'un de l'autre.

5. Dispositif selon l'une des revendications 1 à 4, caractérisé en ce que le rayon lumineux du premier laser (4) peut être séparé par un séparateur de lumière (16) produisant au moins un premier et un second rayonnement de lumière dans la première plage de longueurs d'ondes qui sont guidés au moins partiellement de manière séparée et qui sont ensuite dirigés sur l'objet du support (22) d'objets.

6. Dispositif selon la revendication 5, caractérisé en ce que le séparateur de lumière (16) est un séparateur de lumière polarisant produisant un premier rayon lumineux avec une lumière polarisée-s et un second rayon lumineux avec une lumière polarisée-p et établissant la phase entre ces deux rayons lumineux, et en ce que le rapport en pourcentage entre les intensités des rayonnements lumineux respectifs au sein de la première plage de longueurs d'ondes est réglable.

7. Dispositif selon l'une des revendications 1 à 6, caractérisé en ce que les miroirs et les diviseurs de lumière (8,9,20) prévus sur les voies lumineuses des rayons lumineux des premier (4) et second (3) lasers peuvent être toumés et basculés indépendamment des optiques de grossissement.

8. Dispositif selon l'une des revendications 1 à 7, caractérisé en ce que le rayon lumineux de la lumière d'observation peut être focalisé par réglage de l'objectif (21) et/ou du support (22) d'objets le long de l'axe optique (axe-z) passant par l'objet sur le support (22) d'objets et en ce que le lieu d'observation pour le rayon lumineux de la lumière d'observation est réglable dans le plan d'objet (plan x-y) par réglage du support (22) d'objets à l'intérieur du plan objet selon un plan x-y.

9. Dispositif selon l'une des revendications 1 à 8, caractérisé en ce qu'il est prévu un réducteur de rayons sur les voies lumineuses du premier (4) respectivement du second (3) laser, réducteur avec lequel les rayons lumineux dans leurs plages de longueurs d'ondes respective peuvent être diminués de manière continue ou pas à pas avant qu'ils ne soient dirigés sur l'objet du support (22) d'objets.

10. Dispositif selon l'une des revendications 1 à 9, caractérisé en ce que les rayons lumineux dans la première plage de longueurs d'ondes et les rayons lumineux dans la seconde plage de longueurs d'ondes sont dirigés sur leur objet respectif du support (22) d'objets au travers d'un miroir commun (7) et au travers d'un objectif commun (21).

11. Dispositif selon l'une des revendications 1 à 10, caractérisé en ce que les rayons lumineux produits par les dispositifs de génération de rayonnement, ensuite traités, déviés et focalisés sur leurs objets respectifs se situent tous sensiblement dans leur premier plan, en ce que le support (22) d'objets se situe dans un second plan (plan x-y) orthogonal au premier plan et en ce que les miroirs et diviseurs de rayonnement (7,8,9,19,20) d'orientation des rayonnements lumineux individuels sont agencés également sensiblement perpendiculaires au premier plan.

12. Procédé de manipulation, traitement et d'observation de petites particules, notamment de particules biologiques, dans lesquelles les objets sont fixés dans un piège optique dans un support (22) d'objets au moyen d'au moins un premier laser (4) produisant des rayons lumineux dans une première plage de longueurs d'ondes par lequel ces objets sont observés et/ou leurs comportements sont enregistrés par des dispositifs (1,2,23) d'observation et d'enregistrement, et dans lesquelles est utilisé au moins un second laser (3) produisant des rayons lumineux dans une seconde plage de longueurs d'ondes focalisée avec une convergence suffisante pour traiter les petites parties correspondantes dans la zone du support (22) d'objets caractérisé en ce qu'on utilise pour chaque rayon lumineux du premier (4) et second (3) lasers sa propre optique d'agrandissement (12,13 ; 14,15 ; 10,11) qui sont chacune réglable en trois dimensions, notamment dans les trois axes orthogonaux (x, y, z) de telle sorte que les rayons lumineux dans la première plage de longueurs d'ondes, les rayons lumineux dans la seconde plage de longueurs d'ondes et les rayons lumineux d'une lumière d'observation sont réglables dans le plan de l'objet (plan x-y) et peuvent être focalisés dans le sens axial (sens z) chacun de manière indépendante les uns des autres avec des dispositifs optiques séparés et en ce que les rayons lumineux de la première plage de longueurs d'ondes, les rayons lumineux de la seconde plage de longueurs d'ondes et les rayons lumineux de la lumière d'observation peuvent être focalisés au début de la manipulation et de l'observation indépendamment de leurs longueurs d'ondes dans le même plan objets (plan x-y) du support (22) d'objets, focalisation dans laquelle chacun des rayonnements peut être influencé indépendamment des autres afin d'obtenir un mouvement des petites particules dans le support (22) d'objets, afin de pouvoir travailler de manière tout à fait ciblée en une position souhaitée, leur enveloppe tridimensionnelle et dans lequel la focalisation pour la lumière visible d'observation peut être maintenue constante.

13. Procédé selon la revendication 12 caractérisé en ce qu'une petite particule piégée dans le piège optique du premier laser correspondant (4) peut être déplacée
a) par réglage d'au moins un rayon lumineux de la première plage de longueurs d'ondes dans le sens x-y et/ou
b) par réglage du support (22) d'objets dans la direction x-y du plan objets, dans le premier cas a), seules les petites particules piégées peuvent être déplacées, et dans le second cas b) l'ensemble des particules, hormis celles piégés, peuvent être déplacées.

14. Procédé selon les revendications 12 ou 13, caractérisé en ce qu'une petite particule piégée dans le piège optique du premier laser (4) correspondant peut être déplacée
a) par réglage de la situation de focalisation d'au moins un rayon lumineux dans la première zone de longueurs d'ondes et ceci dans le sens-z et/ou
b) par réglage dans le sens-z de l'objectif (21) et/ou du support (22) d'objets, réglages par lesquels dans le premier cas a) les particules piégées sont déplacées hors du plan d'observation choisi et, dans le cas b), les petites particules piégées sont maintenues dans le plan d'observation choisi.

15. Procédé selon l'une des revendications 12 à 14, caractérisé en ce que par utilisation d'au moins deux rayons lumineux séparés dans la première plage de longueurs d'ondes, on obtient une rotation d'une petite particule dans le piège optique lorsque
a) l'un des rayons lumineux reste en sa position de sortie et l'autre rayon lumineux effectue un déplacement dans la direction x-y
b) l'un des rayons lumineux reste en sa situation de sortie et l'autre rayon lumineux effectue un déplacement dans la direction z, ou
c) au moins deux rayons lumineux effectuent des mouvements en sens contraire ou des mouvements d'ampleur différente dans la direction z, ou
d) une combinaison des mouvements selon a), b) et c).

16. Procédé selon l'une des revendications 12 à 15, caractérisé en ce que le traitement des petites particules avec des rayons lumineux de la deuxième plage de longueurs d'ondes est effectué dans un plan x-y quelconque du support (22) d'objets, le plan d'observation pouvant être ce même plan ou un autre plan parallèle à ce premier.

17. Procédé selon l'une des revendications 12 à 16, caractérisé en ce qu'on utilise des rayons lasers visibles ou infrarouges pour la fixation des petites particules dans le puits optique et des rayons lasers UV, notamment des rayons lasers UV pulsés, pour le traitement des petites particules.

18. Procédé selon l'une des revendications 12 à 17, caractérisé en ce que l'ensemble des rayons lumineux peut simultanément être dirigé sur l'objet correspondant du support (22) d'objets par le même objectif (21).

19. Procédé selon l'une des revendications 12 à 18, caractérisé en ce que l'ensemble des rayons lumineux est réglable en intensité et/ou peuvent être allumés ou éteints indépendamment les uns des autres pour conduire le traitement et/ou l'observation.
